(19) Europäisches Patentamt
European Patent Office
Office européen des brevets

(11) Veröffentlichungsnummer: **0 012 957**
**B1**

(12) # EUROPÄISCHE PATENTSCHRIFT

(45) Veröffentlichungstag der Patentschrift: **16.09.81**

(51) Int. Cl.³: **C 12 Q 1/00,** C 12 Q 1/34

(21) Anmeldenummer: **79105184.0**

(22) Anmeldetag: **14.12.79**

(54) **Diagnostisches Mittel zum Nachweis proteolytischer Enzyme sowie als Chromogene hierfür geeignete Indoxyl- bzw. Thioindoxylester, Verfahren zu deren Herstellung sowie deren Verwendung zur Herstellung diagnostischer Mittel.**

(30) Priorität: **20.12.78 DE 2854987**

(43) Veröffentlichungstag der Anmeldung: **09.07.80 Patentblatt 80/14**

(45) Bekanntmachung des Hinweises auf die Patenterteilung: **16.09.81 Patentblatt 81/37**

(84) Benannte Vertragsstaaten: **AT BE CH DE FR GB IT LU NL SE**

(56) Entgegenhaltungen:
**BE-A-615 395**
**GB-A-1 128 371**
**CHEMICAL ABSTRACTS, Band 69, Nr. 1, 1. Juli 1968, Zusammenfassung Nr. 255m, Seite 22, Columbus, Ohio, US**
**G.G. GUILBAULT et al.: «N-Methylindoxyl esters as substrates for cholinesterase»**
**CHEMICAL ABSTRACTS, Band 71, Nr. 7, 18. August 1969, Zusammenfassung Nr. 27522z, Seite 19 Columbus, Ohio, US,**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Berger, Dieter, Dr.rer.nat., Bensheimer Strasse 45, D-6806 Viernheim (DE)**
Erfinder: **Braun, Franz, Dr.rer.nat., Ringstrasse 14, D-6149 Rimbach/Odw. (DE)**
Erfinder: **Güthlein, Werner, Dr.rer.nat., Im Sennteich 31, D-6800 Mannheim/Neckarau (DE)**
Erfinder: **Kuhr, Manfred, Dr.rer.nat., Werthmannweg 23, D-6800 Mannheim 31 (DE)**
Erfinder: **Werner, Wolfgang, Dr.rer.nat., Meissener Weg 39, D-6800 Mannheim 42 (DE)**

(56) Entgegenhaltungen:
**G.G. GUILBAULT et al.: «N-Methyl indoxyl esters as fluorometric substrates for lipase»**
**CHEMICAL ABSTRACTS, Band 80, Nr. 9, 4. März 1974, Zusammenfassung Nr. 45560a, Seite 166, Columbus, Ohio, US, M. RODLER et al.: «Differentiation of bacteria by indoxyl butyrate paper strip method»**

**EP 0 012 957 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Diagnostisches Mittel zum Nachweis proteolytischer Enzyme sowie als Chromogene hierfür geeignete Indoxyl- bzw. Thioindoxylester, Verfahren zu deren Herstellung sowie deren Verwendung zur Herstellung diagnostischer Mittel

Der Nachweis von Leukozyten in Körperflüssigkeiten, insbesondere im Urin, nimmt eine hervorragende Stelle in der Diagnostik der Erkrankungen der Niere und des Urogenitaltraktes ein.

Bisher wird dieser Nachweis geführt durch mühsames Auszählen der Leukozyten im nicht zentrifugierten Harn oder im Harnsediment.

Beiden Methoden ist naturgemäss gemeinsam, dass nur intakte Leukozyten erfasst werden. Andererseits ist bekannt, dass die Geschwindigkeit der Leukozyten-Lyse je nach Harnmilieu enormen Schwankungen unterworfen ist; so ist z.B. in stark alkalischen Harnen mit einer Leukozyten-Halbwertszeit von nur 60 Minuten zu rechnen. Zu niedrige Leukozytenzahlen bzw. bei längeren Harnstandzeiten sogar falschnegative Befunde sind die Folge.

Vom Lyse-Fehler abgesehen, liefert die quantitative mikroskopische Bestimmung der Leukozyten im nicht zentrifugierten, homogenisierten Harn in der Zählkammer recht zuverlässige Werte. In der Praxis wird diese Methode jedoch nur selten angewandt, da sie mühevoll, ermüdend und zeitraubend ist und den Einsatz geschulten Personals bedingt.

Die überwiegende Mehrzahl der Leukozytenbestimmungen im Harn werden in der medizinischen Praxis nach der sogenannten Gesichtsfeldmethode im Harnsediment durchgeführt. Hierzu muss zunächst das Untersuchungsgut (Sediment) durch Zentrifugieren gewonnen werden. Dabei werden jedoch auch andere Bestandteile des Harnes angereichert, die – wie z.B. Salze und Epithelzellen – die mikroskopische Auszählung der Leukozyten beträchtlich erschweren können. Schwankender Sedimentgehalt, Inhomogenitäten des Sedimentes, sowie womöglich unterschiedliche mikroskopische Vergrösserungen oder unterschiedliche optische Ausstattung der Mikroskope führen dazu, dass die hier übliche Angabe über die Anzahl der Leukozyten pro mikroskopischem Gesichtsfeld mit Fehlern von mehreren hundert Prozent behaftet sein kann.

Aufgabe der vorliegenden Erfindung war es daher, ein diagnostisches Mittel bereitzustellen, mit dem die Leukozyten in Körperflüssigkeiten auf einfache und leicht zu handhabende Weise sowie möglichst schnell und vollständig nachgewiesen werden können.

Als Nachweisprinzip für einen solchen Leukozytentest bietet sich eine enzymatische Reaktion an, da die Leukozyten ein breitgefächertes Enzymspektrum besitzen.

In dem US-Patent 30 87 794 ist bereits ein Leukozytennachweis beschrieben und beansprucht, der über die in den granulozytären Leukozyten vorhandene peroxidatische Aktivität geführt wird. Ein saugfähiger Träger, der mit Wasserstoffperoxid und einem organischen Indikator, beispielsweise o-Tolidin, imprägniert ist, zeigt die Anwesenheit von Leukozyten durch die Bildung eines farbigen Oxidationsproduktes an. Ein solcher Test besitzt jedoch entscheidende Nachteile: Zum einen besitzen peroxidatische Reaktionen unter Verwendung von o-Tolidin ganz allgemein gegenüber reduzierenden Substanzen im Harn, wie z.B. gegenüber Ascorbinsäure, eine erhebliche Störanfälligkeit. Darüber hinaus finden sich in mehreren Literaturstellen (s. z.B. L. Mettler, Med. Welt 23, 399 [1972]) Hinweise auf die Instabilität der Leukozytenperoxidase im Harnmilieu, die zu falschnegativen Befunden Anlass gibt. Noch gravierender ist die zu erwartende mangelhafte Selektivität dieses Testes gegenüber Erythrozyten.

Seit einigen Jahren haben in der histo- und zytochemischen Enzymologie Nachweismethoden ihren festen Platz, die auf der esterolytischen Aktivität der in den zu bestimmenden Systemen vorhandener Enzyme beruhen (vgl. z.B. A.G.E. Pearse, Histochemistry, Theoretical and Applied). Im Prinzip werden dabei farblose oder schwach gefärbte Ester eingesetzt, die durch die enzymatische Spaltung zumeist in eine farblose Säure- und eine ebenfalls farblose Alkohol-(Phenol)-Komponente zerfallen. Letztere wird dann in einer der enzymatischen Verseifung folgenden Reaktion zu farbigen Produkten umgesetzt (z.B. Kupplung mit Diazoniumsalzen oder oxidative Reaktionen).

So beschreiben beispielsweise F. Schmalzl und H. Braunsteiner in Klin. Wschr. 46, 642 (1968) einen spezifischen zytochemischen Leukozytenesterase-Nachweis mit Naphthol-AS-D-chloracetat als Substrat und einem Diazoniumsalz zur Bildung der farbigen Azo-Verbindung.

Für ein diagnostisches Mittel zum schnellen und einfachen Nachweis von Leukozyten in Körperflüssigkeiten, wie z.B. im Harn, erweisen sich Zwei-Komponenten-Systeme dieser Art als nicht geeignet, da bekanntlich viele im Harn vorkommende Verbindungen, wie Urobilinogen, Stercobilinogen, Bilirubin u.a., mit Diazoniumsalzen reagieren. Darüber hinaus ist dieser Nachweis viel zu unempfindlich. Beispielsweise zeigen Proben mit 5000 Leukozyten/µl keine Reaktion.

In dem britischen Patent 11 28 371 wird nun ein diagnostisches Mittel zum Nachweis hydrolytischer Enzyme in Körperflüssigkeiten beschrieben und beansprucht. Hierbei wird ein saugfähiger Träger mit farblosen Indoxyl- oder Thioindoxyl-Estern und gegebenenfalls einem Puffer und einem Oxidationsmittel getränkt. Bei Anwesenheit hydrolytischer Enzyme entstehen aus den Estern freies Indoxyl oder Thioindoxyl und hieraus durch Einwirkung von Luftsauerstoff oder Oxidationsmittel tiefgefärbtes Indigo oder Thioindigo. Die in diesem Patent beanspruchten Verbindungen sind für einen Leukozytentest nicht brauchbar, da sie selbst mit 10 000 Leukozyten/µl keine Reaktion zeigen.

So ist bis heute kein Teststreifen im Handel, der

es gestattet, einfach und schnell Leukozyten nachzuweisen, obwohl der Leukozytennachweis im Harn zu der am häufigsten durchgeführten klinischen Untersuchung gehört.

Überraschenderweise wurde nun gefunden, dass man stabile und schnell anzeigende diagnostische Mittel, mit denen Leukozyten gut in Körperflüssigkeiten nachzuweisen sind, erhält, wenn als Substrate zum Nachweis der in den neutrophilen Leukozyten-Granulozyten vorkommenden Esterasen (Proteasen), Indoxyl- oder Thioindoxyl-Aminosäureester oder -Peptidester verwendet werden. Darüber hinaus zeigte sich, dass sich diese Substrate auch ausgezeichnet zum allgemeinen Nachweis proteolytischer Enzyme, wie z.B. von Elastase, Chymotrypsin oder Trypsin in rein wässrigen Lösungen oder auch in Körperflüssigkeiten, wie z.B. Plasma, Serum, Liquor, Pankreassekret oder wässrigen Stuhlextrakten, eignen.

Gegenstand der vorliegenden Erfindung ist daher ein diagnostisches Mittel zum Nachweis proteolytischer Enzyme, insbesondere zum Nachweis der in den Leukozyten vorhandenen Proteasen in Körperflüssigkeiten, bestehend aus einem saugfähigen Träger, einer Filmschicht, einer Pulvermischung, einem Lyophilisat, einer Lösung oder einer Reagenztablette, enthaltend ein oder mehrere Chromogene und jeweils übliche Zusatzstoffe, dadurch gekennzeichnet, dass als Chromogene Indoxyl- und/oder Thioindoxyl-Aminosäureester und/oder -Peptidester der allgemeinen Formel I

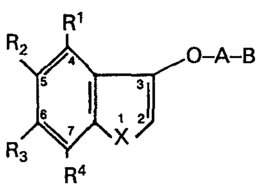

(I),

in der

$R_1$, $R_2$, $R_3$, $R_4$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,

X ein Schwefelatom oder eine gegebenenfalls durch einen niederen Alkyl-, einen Aryl-, einen Aralkyl-oder einen Acylrest substituierte Iminogruppe,

A einen Aminosäure- oder einen Peptidrest,

B eine in der Peptidchemie übliche oder davon abgeleitete Stickstoffschutzgruppe bedeutet,

eingesetzt werden.

Ein weiterer Gegenstand der vorliegenden Erfindung ist die Verwendung von Indoxyl- und/ oder Thioindoxyl-Aminosäureestern und/oder -Peptidestern der allgemeinen Formel I zur Herstellung von diagnostischen Mitteln zum Nachweis proteolytischer Enzyme, insbesondere der in den Leukozyten vorhandenen Proteasen in Körperflüssigkeiten.

Sämtliche Indoxyl- und Thioindoxyl-Aminosäureester und -Peptidester der allgemeinen Formel I sind neue Verbindungen.

Gegenstand der vorliegenden Erfindung sind daher ferner die Indoxyl- und Thioindoxyl-Aminosäureester und -Peptidester der allgemeinen Formel I sowie Verfahren zu deren Herstellung.

Die Herstellung der neuen Indoxyl- und Thioindoxyl-Aminosäureester und -Peptidester der allgemeinen Formel I kann nach an sich aus der Peptidchemie her bekannten Methoden erfolgen.

Vorzugsweise werden in an sich bekannter Weise die entsprechenden Indoxyl- oder Thioindoxyl-Verbindungen der allgemeinen Formel II

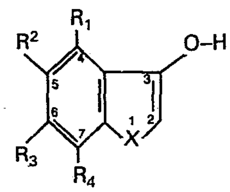

(II),

in der $R_1$, $R_2$, $R_3$, $R_4$ und X die oben angegebene Bedeutung haben, mit Aminosäuren und Peptiden der allgemeinen Formel III

HO–A–B (III),

in der A und B die oben angegebene Bedeutung haben, beziehungsweise mit geeigneten reaktiven Derivaten davon, umgesetzt.

Als reaktive Derivate werden z.B. die Säurechloride, beziehungsweise die bei der Peptidsynthese üblicherweise verwendeten Mischanhydride, beispielsweise mit Chlorameisensäureethylester oder Aktiv-Ester eingesetzt.

Die Indoxyl- und Thioindoxyl-Verbindungen der allgemeinen Formel II, sowie die Aminosäuren und Peptide der allgemeinen Formel III sind bekannte Substanzen (vgl. beispielsweise P. Friedländer, Fortschritte der Teerfarbenfabrikation und verwandter Industriezweige, Bd. 3–20 bzw. Houben-Weyl, Methoden der organischen Chemie, Bd. 15/1), oder können in Analogie zu bekannten Verbindungen hergestellt werden.

Unter Halogen in der Definition von $R_1$, $R_2$, $R_3$ und $R_4$ ist Fluor, Chlor, Brom und Iod, vorzugsweise Chlor und Brom zu verstehen.

Die «niedere Alkoxygruppe» in der Definition von $R_1$, $R_2$, $R_3$ und $R_4$, sowie die «niedere Alkylgruppe» von $R_1$, $R_2$, $R_3$, $R_4$ und X enthalten 1 bis 5, vorzugsweise 1 bis 3 Kohlenstoffatome, wobei die Methoxy- und die Methylgruppe ganz besonders bevorzugt sind.

Unter einer «Aralkoxygruppe» in der Definition von $R_1$, $R_2$, $R_3$ und $R_4$, sowie einer «Aralkylgruppe» in der Definition von $R_1$, $R_2$, $R_3$, $R_4$ und X sind z.B. durch Oxy-nieder-Alkyl- bzw. niedere Alkylreste substituierte Phenyl- und Naphthylgruppen zu verstehen, wobei der Alkylrest 1 bis 5, vorzugsweise 1 bis 3 Kohlenstoffatome enthält. Besonders bevorzugt sind der Benzyloxy- und der Benzylrest.

Als «niedere Acylaminogruppe» von $R_1$, $R_2$, $R_3$ und $R_4$ kommen die Amidgruppierungen der niederen aliphatischen Carbonsäuren mit 1 bis 5, vorzugsweise 1 bis 3 Kohlenstoffatomen, in Frage. Besonders bevorzugt ist der Acetylaminorest.

Als «Acylrest» in der Definition von X kommen die Reste aliphatischer Carbonsäuren mit 1 bis 5, vorzugsweise 1 bis 3 Kohlenstoffatomen, oder auch aromatischer Carbonsäuren, wie beispielsweise der Benzoe- oder Naphthoesäuren, in Frage. Besonders bevorzugt sind Acetyl- und Benzoylreste.

Unter einem «Arylrest» in der Definition von $R_1$, $R_2$, $R_3$, $R_4$ und X sind vorzugsweise die Phenyl- oder die Naphthylgruppe zu verstehen.

Als «Aminosäurerest» in der Definition von A kommen vorzugsweise die Reste der natürlichen α-Aminosäuren in ihrer L- oder D-Form oder auch in ihrer racemischen Form, in Frage. Besonders bevorzugt sind die Reste von Glycin, Alanin, Valin, Leucin, Isoleucin, Phenylalanin und Tyrosin. Gegebenenfalls vorhandene freie Hydroxygruppen können acyliert, vorzugsweise acetyliert, sein.

Unter einem «Peptidrest» in der Definition von A sind z.B. Di-, Tri-, Tetra- und Pentapeptide, vorzugsweise Di- und Tripeptide, zu verstehen, wobei als Aminosäure-Komponenten vorzugsweise die oben erwähnten Aminosäuren Verwendung finden.

Als «in der Peptidchemie übliche Stickstoffschutzgruppe» in der Definition von B sind z.B. Acyl-, Oxycarbonyl-, Thiocarbonyl-, Sulfonyl-, Sulfenyl-, Vinyl-, Cyclohexenyl-, Phosphoryl- oder Carbamoyl-Gruppen zu verstehen.

Die erfindungsgemäss als Chromogene verwendeten Indoxyl- oder Thioindoxyl-Aminosäureester und -Peptidester der allgemeinen Formel I werden in Konzentrationen von $10^{-4}$ bis 1 mol/Liter, vorzugsweise $10^{-3}$ bis $10^{-1}$ mol/Liter Imprägnierlösung, Beschichtungsmasse oder zu untersuchender Flüssigkeit eingesetzt.

Ein weiterer Bestandteil des diagnostischen Mittels zum Nachweis proteolytischer Enzyme und insbesondere der Leukozyten-Proteasen ist ein geeignetes Puffersystem. Hierzu kommen z.B. Phosphat-, Borat-, Barbiturat-, Tris-(hydroxymethyl)-aminomethan-(=Tris-)-, 2-Amino-2-methyl-propandiol-1,3-(=Amediol)- oder Aminosäure-Puffer in Frage, wobei pH-Wert und Kapazität so gewählt werden müssen, dass sich in der Messlösung bzw. auf dem Teststreifen ein pH-Wert von 6–10, vorzugsweise von 7–9, einstellt.

Weiterhin kann man bei der Herstellung des erfindungsgemässen diagnostischen Mittels zum Nachweis proteolytischer Enzyme, insbesondere der Leukozyten-Proteasen in Körperflüssigkeiten zusätzlich Oxidationsmittel verwenden, um die bei der enzymatischen Reaktion primär entstandenen Indoxyl- bzw. Thioindoxyl-Verbindungen zu den gefärbten Indigo- bzw. Thioindigo-Substanzen umzusetzen. Diese Oxidationsmittel, wie z.B. Kalium-hexacyanoferrat-III, Kaliumbromat, Kaliumchromat, Phenazin-methosulfat oder Tetrazoliumsalze werden in Konzentrationen von $10^{-4}$ bis 1 mol/Liter, vorzugsweise $10^{-3}$ bis $10^{-1}$ mol/Liter Imprägnierlösung, Beschichtungsmasse oder zu untersuchender Flüssigkeit eingesetzt.

Ein weiterer Bestandteil eines diagnostischen Mittels für den Nachweis proteolytischer Enzyme, insbesondere der Leukozyten-Proteasen, kann ein Netzmittel sein, da hierdurch etwas verkürzte Reaktionszeiten und z.T. brillantere Farben erzielt werden. Vorzugsweise werden nichtionogene aber auch amphotere, kationen- oder anionenaktive Netzmittel in Konzentrationen von 0.05–2%, vorzugsweise 0.1–1%, eingesetzt.

Zur Herstellung des erfindungsgemässen Mittels werden z.B. saugfähige Träger, vorzugsweise Filterpapier, Cellulose oder Kunstfaservliese, mit Lösungen der erforderlichen, üblicherweise zur Herstellung von Teststreifen verwendeten Reagenzien (Substrat, Puffer, gegebenenfalls Netzmittel, Oxidationsmittel etc.) in leichtflüchtigen Lösungsmitteln, wie z.B. Wasser, Methanol, Ethanol oder Aceton, imprägniert. Dies geschieht zweckmässig in zwei getrennten Schritten: Zunächst wird mit einer wässrigen Lösung imprägniert, die den Puffer und andere wasserlösliche Zusatzstoffe enthält. Danach wird mit einer Lösung der Protease-Substrate der allgemeinen Formel I imprägniert. In speziellen Fällen kann auch die umgekehrte Imprägnierfolge angewandt werden.

Die fertigen Testpapiere können als solche verwendet werden oder in an sich bekannter Weise an Griffen angeklebt oder vorzugsweise zwischen Kunststoffen und feinmaschigen Netzwerken gemäss DBP 21 18 455 eingesiegelt werden.

Zur Herstellung filmbeschichteter Teststreifen werden sämtliche Reagenzien in die Lösung oder Dispersion einer filmbildenden Substanz, wie z.B. Polyvinylester oder Polyamid, eingetragen und homogen vermischt. Das Gemisch wird in dünner Schicht auf einen Kunststoffträger gestrichen und getrocknet. Die erfindungsgemässen filmbeschichteten Teststreifen werden nach dem Trocknen geschnitten und können als solche verwendet werden oder in an sich bekannter Weise an Griffen angeklebt werden oder z.B. zwischen Kunststoffen und feinmaschigen Netzwerken gemäss DBP 21 18 455 eingesiegelt werden.

Das erfindungsgemässe diagnostische Mittel zum Nachweis proteolytischer Enzyme, insbesondere der Leukozyten-Proteasen, in Form von Pulvermischungen oder Reagenztabletten lässt sich herstellen, indem die oben angeführten Bestandteile des Testes mit üblichen galenischen Zusatzstoffen versetzt und granuliert werden. Zusatzstoffe dieser Art sind z.B. Kohlenhydrate, wie z.B. Mono-, Oligo- oder Polysaccharide, oder Zuckeralkohole, wie z.B. Mannit, Sorbit oder Xylit, oder andere lösliche inerte Verbindungen, wie Polyäthylenglykole oder Polyvinylpyrrolidon. Die Pulvermischungen oder Reagenztabletten weisen im allgemeinen ein Endgewicht von ungefähr 50–200 mg, vorzugsweise 50–80 mg, auf.

Zur Herstellung von Lyophilisaten im Gesamtgewicht von jeweils etwa 5–20 mg, vorzugsweise etwa 10 mg, wird eine Lösung gefriergetrocknet, die neben sämtlichen für den Test benötigten Reagenzien übliche Gerüstbildner, wie z.B. Polyvinylpyrrolidon, und evtl. weitere Füllstoffe, wie z.B. Mannit, Sorbit oder Xylit, enthält.

Das erfindungsgemässe diagnostische Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z.B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit proteolytischer Enzyme, insbesondere der Leukozyten-Proteasen, rasch und einfach über eine Farbbildung nachzuweisen, die visuell oder photometrisch, z.B. remissionsphotometrisch oder in der Küvette, beurteilt werden kann. Da die Aktivität der Leukozyten-Proteasen pro Zelle als eine im wesentlichen konstante Grösse angesehen werden kann, lässt sich aus der Intensität der Farbbildung die Leukozytenkonzentration der untersuchten Körperflüssigkeit ermitteln. Dabei werden mit dem erfindungsgemässen diagnostischen Mittel sowohl intakte als auch lysierte Leukozyten erfasst, da die Aktivität der Leukozyten-Proteasen auch nach der Lyse der Leukozyten voll erhalten bleibt. Ein Lysefehler tritt folglich nicht auf.

Beispiel 1

Filterpapier (z.B. Schleicher + Schüll 23 SL) wird nacheinander mit den folgenden Lösungen imprägniert und dann bei 60 °C getrocknet.

Lösung 1

| | |
|---|---|
| Di-natrium-tetraborat-decahydrat | 1.91 g |
| Wasser, dest. | ca. 30 ml |
| Lösung mit 0.1 N Salzsäure auf einen pH-Wert von 8.0 einstellen, | |
| Wasser, dest., ad | 100.0 ml |

Lösung 2

| | |
|---|---|
| 3-[N-(Benzyloxycarbonyl)-L-alanyl-oxy-]-indol | 33.8 mg |
| Aceton, ad | 100.0 ml |

Man erhält ein farbloses Testpapier, welches sich beim Eintauchen in leukozytenhaltige Harne je nach Leukozytenkonzentration helltürkis bis blau verfärbt. Es lassen sich nachweisen:

5000 Leukozyten/µl Harn in ca. 2 Minuten
1000 Leukozyten/µl Harn in ca. 6 Minuten
500 Leukozyten/µl Harn in ca. 10 Minuten
200 Leukozyten/µl Harn in ca. 15 Minuten.

Die Empfindlichkeit des Testes liegt bei etwa 200 Leukozyten/µl. Die Beurteilung kann auch remissionsphotometrisch bei 620 nm vorgenommen werden.

Testpapiere mit ähnlichen Eigenschaften (Empfindlichkeiten: 200–2 000 Leukozyten/µl) erhält man, wenn man anstelle von 3-[N-(Benzyloxycarbonyl)-L-alanyloxy]-indol die folgenden Substrate einsetzt, wobei sich – wenn nicht besonders erwähnt – ebenfalls helltürkise bis blaue Verfärbungen der sonst farblosen Testpapiere beim Eintauchen in leukozytenhaltige Harne beobachten lassen:

---

1.1. 3-[N-(Benzyloxycarbonyl)-L-alanyloxy]-4-methyl-indol
1.2. 3-[N-(Benzyloxycarbonyl)-L-alanyloxy]-5-methyl-indol
1.3. 3-[N-(Benzyloxycarbonyl)-L-alanyloxy]-6-methyl-indol
1.4. 3-[N-(Benzyloxycarbonyl)-L-alanyloxy]-7-methyl-indol
1.5. 3-[N-(Benzyloxycarbonyl)-L-alanyloxy]-4,7-dimethyl-indol
1.6. 3-[N-(Benzyloxycarbonyl)-L-alanyloxy]-4-chlor-indol
1.7. 3-[N-(Benzyloxycarbonyl)-L-alanyloxy]-5-brom-indol
1.8. 3-[N-(Benzyloxycarbonyl)-L-alanyloxy]-6-chlor-indol
Verfärbung: farblos nach purpur
1.9. 3-[N-(Toluol- 4'-sulfonyl)-L-alanyloxy]-4-chlor-5-brom-indol
1.10. 3-[N-(Toluol-4'-sulfonyl)-L-alanyloxy]-4,5,7-trichlor-indol
1.11. 3-[N-(Toluol-4'-sulfonyl)-L-alanyloxy]-4-chlor-5-brom-7-methyl-indol
1.12. 3-[N-(Toluol-4'-sulfonyl)-L-alanyloxy]-5-hydroxy-indol
1.13. 3-[N-(Benzyloxycarbonyl)-L-alanyloxy]-5-methoxy-indol
1.14. 3-[N-(Toluol-4'-sulfonyl)-L-alanyloxy]-5-benzyloxy-indol
1.15. 3-[N-(Toluol-4'-sulfonyl)-L-alanyloxy]-4-carboxy-indol
1.16. 3-[N-(Toluol-4'-sulfonyl)-L-alanyloxy]-4-benzyloxycarbonyl-indol
1.17. 3-[N-(Toluol-4'-sulfonyl)-L-alanyloxy]-5-(carboxy-methoxy)-indol
1.18. 3-[N-(Toluol-4'-sulfonyl)-L-alanyloxy]-5-(benzyloxycarbonyl-methoxy)-indol
1.19. 3-[N-(Toluol-4'-sulfonyl)-L-alanyloxy]-6-nitro-indol
1.20. 3-[N-(Toluol-4'-sulfonyl)-L-alanyloxy]-6-acetylamino-indol
1.21. 3-[N-(Toluol-4'-sulfonyl)-L-alanyloxy]-benzo[g]-indol
Verfärbung: farblos nach grün.
1.22. 1-Methyl-3-[N-(benzyloxycarbonyl)-L-alanyloxy]-indol
Verfärbung: farblos nach grün.
1.23 1-Benzyl-3-[N-(toluol-4'-sulfonyl)- L-alanyloxy]-indol
Verfärbung: farblos nach grün.
1.24. 1-Phenyl-3-[N-(toluol-4'-sulfonyl)-L-alanyloxy]-indol
Verfärbung: farblos nach blaugrün.
1.25 1-Acetyl-3-[N-(toluol-4'-sulfonyl)-L-alanyloxy]-indol
Verfärbung: farblos nach rot.

1.26. 1-Benzoyl-3-[N-(toluol-4'-sulfonyl)-L-alanyloxy]-indol
Verfärbung: farblos nach violett.
1.27. 3-[N-(Benzyloxycarbonyl)-glycyloxy]-indol
1.28. 3-[N-(Toluol-4'-sulfonyl)-glycyloxy]-indol
1.29. 3-[N-(Toluol-2'-sulfonyl)-L-alanyloxy]-indol
1.30. 3-[N-(Toluol-3'-sulfonyl)-L-alanyloxy]-indol
1.31. 3-[N-(Toluol-4'-sulfonyl)-L-alanyloxy]-indol
1.32. 3-[N-(Toluol-4'-sulfonyl)-D-alanyloxy]-indol
1.33. 3-[N-(Benzyloxycarbonyl)-D,L-alanyloxy]-indol
1.34. 3-[N-Benzyloxycarbonyl)-L-valyloxy]-indol
1.35. 3-[N-(Toluol-4'-sulfonyl)-L-valyloxy]-indol
1.36. 3-[N-(Benzyloxycarbonyl)-L-leucyloxy]-indol
1.37. 3-[N-(Benzyloxycarbonyl)-L-isoleucyloxy]-indol
1.38. 3-[N-(Benzyloxycarbonyl)-L-phenylalanyloxy]-indol
1.39. 3-[N-(Toluol-4'-sulfonyl)-L-phenylalanyloxy]-indol
1.40. 3-[N-Acetyl-L-tyrosyloxy]-indol
1.41. 3-[N-Benzoyl-L-tyrosyloxy]-indol
1.42. 3-[N-(Benzyloxycarbonyl)-L-tyrosyloxy]-indol
1.43. 3-[N-(Toluol-4'-sulfonyl)-L-tyrosyloxy]-indol
1.44. 3-[N-(Toluol-4'-sulfonyl)-O-acetyl-L-tyrosyloxy]-indol

1.45. 3-[N-(Benzyloxycarbonyl)-L-alanyl-L-alanyloxy]-indol
1.46. 3-[N-(Toluol-4'-sulfonyl)-D-alanyl-L-alanyloxy]-indol
1.47. 3-[N-(Benzyloxycarbonyl)-L-alanyl-L-alanyl-L-alanyloxy]-indol
1.48. 3-[N-(Toluol-4'-sulfonyl)-D-alanyl-D-alanyl-L-alanyloxy]-indol
1.49. 3-[N-Formyl-L-alanyloxy]-indol
1.50. 3-[N-Acetyl-L-alanyloxy]-indol
1.51. 3-[N-Succinyl-L-alanyloxy]-indol
1.52. 3-[N-Benzoyl-D,L-alanyloxy]-indol
1.53. 3-[N-Phthaloyl-L-alanyloxy]-indol
1.54. 3-[N-(Ethoxycarbonyl)-L-alanyloxy]-indol
1.55. 3-[N-(tert.-Butyloxycarbonyl)-L-alanyloxy]-indol
1.56. 3-[N-(3',6'-Dioxa-n-heptyloxycarbonyl)-L-alanyloxy]-indol
1.57. 3-[N-(Cyclohexyloxycarbonyl)-L-alanyloxy]-indol
1.58. 3-[N-(Phenyloxycarbonyl)-L-alanyloxy]-indol
1.59. 3-[N-(4'-Methyl-benzyloxycarbonyl)-L-alanyloxy]-indol
1.60. 3-[N-(4'-Methoxy-benzyloxycarbonyl)-L-alanyloxy]-indol
1.61. 3-[N-(4'-Nitro-benzyloxycarbonyl)-L-alanyloxy]-indol
1.62. 3-[N-(N'-⟨Piperidino⟩-oxycarbonyl)-L-alanyloxy]-indol
1.63. 3-[N-(Furyl-⟨2'⟩-methoxycarbonyl)-L-alanyloxy]-indol
1.64. 3-[N-(Thienyl-⟨2'⟩-methoxycarbonyl)-L-alanyloxy]-indol
1.65. 3-[N-(Benzylthiocarbonyl)-L-alanyloxy]-indol
1.66. 3-[N-(Methansulfonyl)-L-alanyloxy]-indol
1.67. 3-[N-(Benzylsulfonyl)-L-alanyloxy]-indol
1.68. 3-[N-(Benzolsulfonyl)-L-alanyloxy]-indol
1.69. 3-[N-(4'-Brom-benzolsulfonyl)-L-alanyloxy]-indol
1.70. 3-[N-(4'-Nitro-benzolsulfonyl)-L-alanyloxy]-indol
1.71. 3-[N-(4'-Dimethylamino-benzolsulfonyl)-L-alanyloxy]-indol
1.72. 3-[N-(4'-Acetylamino-benzolsulfonyl)-L-alanyloxy]-indol
1.73. 3-[N-(4'-n-Butyl-benzolsulfonyl)-L-alanyloxy]-indol
1.74. 3-[N-(4'-tert.-Butyl-benzolsulfonyl)-L-alanyloxy]-indol
1.75. 3-[N-(4'-n-Octyl-benzolsulfonyl)-L-alanyloxy]-indol
1.76. 3-[N-(4'-Hydroxy-benzolsulfonyl)-L-alanyloxy]-indol
1.77. 3-[N-(4'-Methoxy-benzolsulfonyl)-L-alanyloxy]-indol
1.78. 3-[N-(4'-Benzyloxy-benzolsulfonyl)-L-alanyloxy]-indol

1.79. 3-[N-(4'-⟨2''-Hydroxy-ethoxy⟩-benzolsulfonyl)-L-alanyloxy]-indol
1.80. 3-[N-(4'-⟨3''-Oxa-5''-hydroxy-n-pentyl-oxy⟩-benzolsulfonyl)-L-alanyloxy]-indol
1.81. 3-[N-(4'-⟨3'',6''-Di-oxa-n-heptyloxy⟩-benzolsulfonyl)-L-alanyloxy]-indol
1.82. 3-[N-( 4'-⟨2''-Hydroxy-ethyl⟩-benzol-sulfonyl)-L-alanyloxy]-indol
1.83. 3-[N-(4'-⟨2''-{4'''-Nitro-benzyloxy}-ethyl⟩-benzolsulfonyl)-L-alanyloxy]-indol
1.84. 3-[N-(4'-⟨2''-Chlor-ethyl⟩-benzolsulfo-nyl)-L-alanyloxy]-indol
1.85. 3-[N-(4'-Acetyl-benzolsulfonyl)-L-alanyloxy]-indol
1.86. 3-[N-(4'-Cyano-benzolsulfonyl)-L-alanyloxy]-indol
1.87. 3-[N-(4'-Carboxy-benzolsulfonyl)-L-alanyloxy]-indol
1.88. 3-[N-(4'-Methoxycarbonyl-benzolsulfo-nyl)-L-alanyloxy]-indol
1.89. 3-[N-(4'-Benzyloxycarbonyl-benzolsulfo-nyl)-L-alanyloxy]-indol
1.90. 3-[N-(4'-Carbamoyl-benzolsulfonyl)-L-alanyloxy]-indol
1.91. 3-[N-(4'-⟨Dimethyl-carbamoyl⟩-benzolsulfonyl)-L-alanyloxy]-indol
1.92. 3-[N-(4'-Carboxymethyl-benzolsulfonyl)-L-alanyloxy]-indol

1.93. 3-[N-(4'-Carboxymethoxy-benzolsulfonyl)-L-alanyloxy]-indol
1.94. 3-[N-(4'-Carboxymethylamino-benzolsulfonyl)-L-alanyloxy]-indol
1.95. 3-[N-(4'-⟨Benzyloxycarbonyl-methyl-amino⟩-benzolsulfonyl)-L-alanyloxy]-indol
1.96. 3-[N-(4'-Fluor-benzolsulfonyl)-L-alanyloxy]-indol
1.97. 3-[N-(4'-Fluorsulfonyl-benzolsulfonyl)-L-alanyloxy]-indol
1.98. 3-[N-(4'-Sulfamoyl-benzolsulfonyl)-L-alanyloxy]-indol
1.99. 3-[N-Methyl-N-(toluol-4'-sulfonyl)-L-alanyloxy]-indol
1.100. 3-[N-Acetyl-N-(toluol-4'-sulfonyl)-L-alanyloxy]-indol
1.101. 3-[N-(2',4',6'-Trimethyl-benzolsulfonyl)-L-alanyloxy]-indol
1.102. 3-[N-(Biphenyl-4'-sulfonyl)-L-alanyloxy]-indol
1.103. 3-[N-(Naphthalin-2'-sulfonyl)-L-alanyloxy]-indol
1.104. 3-[N-(4'-Acetylamino-naphthalin-1'-sulfonyl)-L-alanyloxy]-indol
1.105. 3-[N-(5'-Dimethylamino-naphthalin-1'-sulfonyl)-L-alanyloxy]-indol
1.106. 3-[N-(Chinolin-8'-sulfonyl)-L-alanyloxy]-indol
1.107. 3-[N-(Pyridin-3'-sulfonyl)-L-alanyloxy]-indol
1.108. 3-[N-(2'-Nitro-benzolsulfonyl)-L-alanyloxy]-indol
Verfärbung: gelb nach grün.
1.109. 3-[N-(1'-Methyl-2'-benzoyl-vinyl)-L-alanyloxy]-indol
1.110. 3-[N-(5',5'-Dimethyl-3'-oxo-cyclohexen-1'-yl)-L-alanyloxy]-indol
1.111. 3-[N(Diphenylcarbamoyl)-L-alanyloxy]-indol
1.112. 3-[N-(Di-⟨4'-nitrobenzyl⟩-phosphoryl)-L-alanyloxy]-indol
1.113. 3-[N-(Di-⟨4'-brombenzyl⟩-phosphoryl)-L-alanyloxy]-indol
1.114. 3-[N-(Toluol-4'-sulfonyl)-L-alanyloxy]-5-benzyl-indol
1.115. 3-[N-(Toluol-4'-sulfonyl)-L-alanyloxy]-5-phenyl-indol
1.116. 3-[N-(Toluol-4'-sulfonyl)-L-alanyloxy]-5-methoxy-indol

---

Beispiel 2

Filterpapier (z.B. Schleicher + Schüll 23 SL) wird nacheinander mit den folgenden Lösungen imprägniert und dann bei 60 °C getrocknet.

Lösung 1

| | |
|---|---|
| Tris-(hydroxymethyl)-aminomethan | 0.61 g |
| Kalium-hexacyano-ferrat-III | 32.9 mg |
| Wasser, dest. | ca. 30 ml |
| Lösung mit 0.1 N Salzsäure auf einen pH-Wert von 8.0 einstellen, | |
| Wasser, dest., ad. | 100.0 ml |

Lösung 2

| | |
|---|---|
| 3-[N-(Benzyloxycarbonyl)-L-alanyl-oxy]-benzo-[b]-thiophen | 35.5 mg |
| Aceton, ad. | 100.0 ml |

Man erhält ein gelbgefärbtes Testpapier, welches sich beim Eintauchen in leukozytenhaltige Harne rot verfärbt.

Die Empfindlichkeit des Testes liegt bei etwa 1000 Leukozyten/µl Harn.

Die Auswertung kann auch remissionsphotometrisch bei 576 nm durchgeführt werden.

Mit den Substraten des Beispiels 1 werden mit Oxidationsmitteln, wie beispielsweise dem oben verwendeten Kalium-hexacyano-ferrat-III, oder z.B. mit Kaliumbromat, Kaliumchromat, Phenazinmethosulfat oder Tetrazoliumsalzen, Testpapiere erhalten, die gegenüber analogen Testpapieren ohne Oxidationsmittel z.T. geringfügig verkürzte Reaktionszeiten aufweisen.

Beispiel 3

Filterpapapier (z.B. Schleicher + Schüll 23 SL) wird nacheinander mit den folgenden Lösungen imprägniert und dann bei 60 °C getrocknet.

Lösung 1

| | |
|---|---|
| Di-natrium-tetraborat-decahydrat | 1.91 g |
| Wasser, dest. | ca. 30 ml |
| Nonylphenolpolyglykolether | 0.2 g |
| Lösung wird mit 0.1 N Salzsäure auf einen pH-Wert von 8.0 eingestellt, | |
| Wasser, dest., ad. | 100.0 ml |

Lösung 2

| | |
|---|---|
| 3-[N-(Benzyloxycarbonyl)-L-alanyloxy]-indol | 33.8 mg |
| Aceton, ad. | 100.0 ml |

Man erhält ein farbloses Testpapier, welches sich beim Eintauchen in leukozytenhaltige Harne hell-türkis bis blau – je nach Leukozytenkonzentration – verfärbt. Gegenüber der Rezeptur des Beispiels 1 ergeben sich etwas verkürzte Reaktionszeiten und geringfügig brillantere Farben.

Auch mit den anderen Substraten der Beispiele 1 und 2 werden mit Netzmitteln, wie beispielsweise dem oben verwendeten Nonylphenolpolyglykolether (nichtionogen), aber auch z.B. mit Cocosimidazolin-Verbindungen (amphoter) oder Benzyltrimethylammoniumchlorid (kationenaktiv) oder Natriumsulfonato-dodecylbenzol (anionenaktiv), Testpapiere erhalten, die gegenüber analogen Testpapieren ohne Netzmittel z.T. geringfügig verkürzte Reaktionszeiten und etwas brillantere Farben aufweisen.

Beispiel 4

Lösung 1

| | |
|---|---|
| 3-[N-Benzoyl-D,L-alanyloxy]-indol | 154.2 mg |
| Methanol, ad. | 100.0 ml |

Lösung 2

| | |
|---|---|
| Di-natrium-tetraborat-decahydrat | 7.63 g |
| Wasser, dest. | ca. 50 ml |

Lösung mit 1 N Salzsäure auf einen pH-Wert von 8.0 einstellen,

| | |
|---|---|
| Wasser, dest., ad. | 100.0 ml |

Es werden gemischt in einem Reagenzglas:
1 ml Lösung 1
1 ml Lösung 2
2 ml leukozytenhaltiger Harn

Das Gemisch verfärbt sich allmählich – je nach Leukozytenkonzentration – hellgrün bis tiefblau.

Nach etwa 10 Minuten Stehenlassen bei Raumtemperatur wird die Leukozytenkonzentration visuell mit Hilfe von Vergleichsfarben bestimmt oder photometrisch, z.B. in 1 cm-Küvetten bei 620 nm.

Die Empfindlichkeit des Testes liegt bei etwa 200 Leukozyten/µl Harn.

Auch mit den anderen Substraten der Beispiele 1 und 2 lassen sich Reagenzglas- bzw. Küvettenteste mit ähnlichen Empfindlichkeiten (200–1000 Leukozyten/µl Harn) durchführen.

Beispiel 5

Eine Tablette, enthaltend

| | |
|---|---|
| 3-[N-(4'-Carboxymethylamino-benzolsulfonyl)-L-alanyloxy]-indol | 2.0 mg |
| Kaliumdihydrogenphosphat | 0.8 mg |
| Di-natriumhydrogenphosphat-dihydrat | 16.8 mg |
| Mannit, ad. | 70.0 mg |

wird zu 2 ml eines leukozytenhaltigen Harnes in einem Reagenzglas gegeben. Der Harn verfärbt sich allmählich – je nach Leukozytenkonzentration – hellgrün bis tiefblau.

Nach 10 Minuten Stehenlassen bei Raumtemperatur wird die Leukozytenkonzentration visuell mit Hilfe von Vergleichsfarben bestimmt oder photometrisch, z.B. in 1 cm-Mikro-Küvetten bei 620 nm.

Die Empfindlichkeit des Testes liegt bei etwa 200 Leukozyten/µl Harn. Die Reaktionszeit lässt sich deutlich verkürzen, wenn die Inkubation bei 37 °C durchgeführt wird.

Mit den anderen Substraten der Beispiele 1 und 2 lassen sich ähnliche Empfindlichkeiten (200–1000 Leukozyten/µl) erreichen, wobei sich bei schwerlöslichen Substraten die Zugabe organischer Lösungsmittel, wie z.B. Methanol oder Dimethylformamid, empfiehlt.

Beispiel 6

Filterpapier (z.B. Schleicher + Schüll 23 SL) wird nacheinander mit den folgenden Lösungen imprägniert und dann bei 60 °C getrocknet.

Lösung 1

| | |
|---|---|
| Di-natrium-tetraborat-decahydrat | 1.91 g |
| Wasser, dest. | ca. 30 ml |

Lösung wird mit 0.1 N Salzsäure auf einen pH-Wert von 8.0 eingestellt,

| | |
|---|---|
| Wasser, dest., ad. | 100.0 ml |

Lösung 2

| | |
|---|---|
| 3-[N-(Toluol-4'-sulfonyl)-L-tyrosyloxy]-indol | 45.1 mg |
| Aceton, ad. | 100.0 ml |

Man erhält ein farbloses Testpapier, welches sich beim Eintauchen in wässrige Lösungen, die das proteolytische Enzym Chymotrypsin enthalten, blau verfärbt. Es lassen sich so noch Konzentrationen von 0.02 U Chymotrypsin pro ml in ca. 6–7 Minuten nachweisen.

(Die angegebene Enzymaktivität wurde mit N-Acetyl-L-tyrosin-ethylester als Substrat bei 25 °C, pH 7,0 und $\lambda$ = 237 nm bestimmt.)

Auch mit den anderen Substraten der Beispiele 1 und 2 lassen sich – je nach Aminosäure- bzw. Peptidrest – Chymotrypsin oder andere proteolytische Enzyme, wie z.B. Elastase oder Trypsin, in rein wässrigen Lösungen oder auch z.B. in Körperflüssigkeiten, wie z.B. Vollblut, Serum, Liquor, Pankreassekret oder wässrigen Stuhlextrakten, nachweisen.

Beispiel 7

3-[N-(Toluol-4'-sulfonyl)-L-alanyloxy]-indol

Lösung 1

Zur Herstellung des Säurechlorids nach der Einstufen-Methode werden 5.35 g (0.022 mol) N-(Toluol-4-sulfonyl)-L-alanin in 20 ml abs. Dimethylformamid (DMF) gelöst und auf –30 °C abgekühlt. Dann werden unter Rühren und Kühlen 1.76 ml (0.024 mol) Thionylchlorid zupipettiert und das Reaktionsgemisch 30 Minuten im Kältebad bei –30 °C belassen.

Lösung 2

In einen 250 ml-Dreihalskolben, der mit Rührer, Thermometer und einer Gas-Zu- und -Ableitung versehen ist, gibt man nach der vollständigen Entfernung der Luft durch einen schwachen Stickstoffstrom die Lösung von 2.90 g (0.022 mol) Indoxyl (3-Hydroxy-indol) in 40 ml abs. DMF, fügt 9.74 ml abs. Pyridin hinzu und kühlt auf –15 °C ab.

Umsetzung

Man giesst Lösung 1 zu Lösung 2 und rührt unter Sauerstoff- und Wasserausschluss ca. 5 h bei –15 °C, bis mittels DC kein Indoxyl mehr zu erkennen ist.

Zur Aufarbeitung wird das Reaktionsgemisch im Vakuum bei maximal 40–50 °C Badtemperatur eingeengt. Der Rückstand wird in 100 ml Essigester aufgenommen und nacheinander zweimal mit je 30 ml 1 N Zitronensäure, 20 ml Wasser, 50 ml 5-proz. Natriumhydrogencarbonatlösung und 25 ml Wasser gewaschen. Die Essigesterphase wird – nach Trocknen mit Natriumsulfat – im Vakuum eingedampft.

Das so erhaltene Rohprodukt wird säulenchromatographisch an Kieselgel mit einem Toluol-Dioxan-Gemisch (9:1) gereinigt. Nach dem Abdestillieren des Lösungsmittels der gesammelten Fraktionen im Vakuum erhält man durch Anrühren des

Rückstandes in Ether 1.45 g (18,4%) 3-[N-(Toluol-4'-sulfonyl)-L-alanlyolxy]-indol, farblose Kristalle, Schmp. 103 °C; $\alpha_D^{20}$: −56,6°, c = 1% (Methanol).

Als Nebenprodukt lässt sich aus anderen Fraktionen der o.a. säulenchromatographischen Trennung noch 0.89 g (11,3%) 1-[N-(Toluol-4'-sulfonyl)-L-alanyloxy]-3-hydroxy-indol = 1-[N-(Toluol-4'-sulfonyl)-L-alanyloxy]-3-oxo-indolin, aus Essigester: farblose Kristalle, Schmp. 187–188 °C, $\alpha_D^{20}$ : −41.7°, c = 1% (DMF), isolieren.

In analoger Weise erhält man durch Umsetzung der entsprechend substituierten Indoxyl-Verbindungen mit den jeweiligen Aminosäuren die folgenden Substrate, wobei in allen Fällen (ausser 7.23. – 7.27.) als Nebenprodukte die o.a. 1-substituierten 3-Hydroxy-indole gebildet werden:

---

7.1. 3-[N-(Benzyloxycarbonyl)-L-alanyloxy]-indol, farblose Kristalle, Schmp. 123 °C, $\alpha_D^{20}$: −49.0°, c = 1% (Methanol).

7.2. 3-[N-(Benzyloxycarbonyl)-L-alanyloxy]-4-methyl-indol, farblose Kristalle, Schmp. 113 °C, $\alpha_D^{20}$: −44.6°, c = 1% (Methanol).

7.3. 3-[N-(Benzyloxycarbonyl)-L-alanyloxy]-5-methyl-indol, farblose Kristalle, Schmp. 143–144 °C, $\alpha_D^{20}$: −48.2°, c = 1% (Methanol).

7.4. 3-[N-(Benzyloxycarbonyl)-L-alanyloxy]-6-methyl-indol, farblose Kristalle, Schmp. 148 °C, $\alpha_D^{20}$: −49.0°, c = 1% Methanol).

7.5. 3-[N-(Benzyloxycarbonyl)-L-alanyloxy]-7-methyl-indol, farblose Kristalle, Schmp. 146 °C, $\alpha_D^{20}$: −51.2°, c = 1% (Methanol).

7.6. 3-[N-(Benzyloxycarbonyl)-L-alanyloxy]-4,7-dimethyl-indol, farblose Kristalle, Schmp. 121 °C, $\alpha_D^{20}$: −46.3°, c = 1% (Methanol).

7.7. 3-[N-Benzyloxycarbonyl)-L-alanyloxy]-4-chlor-indol, farblose Kristalle, Schmp. 142–143 °C, $\alpha_D^{20}$: −61.4°, c = 1% (Methanol).

7.8. 3-[N-(Benzyloxycarbonyl)-L-alanyloxy]-5-brom-indol, farblose Kristalle, Schmp. 138 °C, $\alpha_D^{20}$: −28.0°, c = 1% (Methanol).

7.9. 3-[N-(Benzyloxycarbonyl)-L-alanyloxy]-6-chlor-indol, farblose Kristalle, Schmp. 170 °C, $\alpha_D^{20}$: −41.3°, c = 1% (Methanol).

7.10. 3[N-(Toluol-4'-sulfonyl)-L-alanyloxy]-4-chlor-5-brom-indol, farblose Kristalle, Schmp. 150–152 °C, $\alpha_D^{20}$: −31.0°, c = 1% (Methanol).

7.11. 3-[N-(Toluol-4'-sulfonyl)-L-alanyloxy]-4,5,7-trichlor-indol

7.12. 3-[N-(Toluol-4'-sulfonyl)-L-alanyloxy]-4-chlor-5-brom-7-methyl-indol schwach beigefarbene Kristalle, Schmp. 143–145 °C, DC: Fertigplatte Kieselgel, (Laufmittel: Toluol-Dioxan 2:1, Detektion: UV, $NH_3$ (Gas), $R_F$-Wert: 0,56)

7.13. 3-[N-(Toluol-4'-sulfonyl)-L-alanyloxy]-5-hydroxy-indol

7.14. 3-[N-(Benzyloxycarbonyl)-L-alanyloxy]-5-methoxy-indol

7.15. 3-[N-(Toluol-4'-sulfonyl)-L-alanyloxy]-5-benzyloxy-indol

7.16. 3-[N-(Toluol-4'-sulfonyl)-L-alanyloxy]-4-carboxy-indol

7.17. 3-[N-(Toluol-4'-sulfonyl)-L-alanyloxy]-4-benzyloxy-carbonyl-indol

7.18. 3-[N-(Toluol-4'-sulfonyl)-L-alanyloxy]-5-(carboxy-methoxy)-indol

7.19. 3-[N-(Toluol-4'-sulfonyl)-L-alanyloxy]-5-(benzyloxy-carbonyl-methoxy)-indol

7.20. 3-[N-(Toluol-4'-sulfonyl)-L-alanyloxy]-6-nitro-indol

7.21. 3-[N-(Toluol-4'-sulfonyl)-L-alanyloxy]-6-acetylamino-indol

7.22. 3-[N-(Toluol-4'-sulfonyl)-L-alanyloxy]-benzo-[g]-indol

7.23. 1-Methyl-3-[N-(benzyloxycarbonyl)-L-alanyloxy]-indol, farblose Kristalle, Schmp. 92 °C, $\alpha_D^{20}$: −49.5°, c = 1% (Methanol).

7.24. 1-Benzyl-3-[N-toluol-4'-sulfonyl)-L-alanyloxy]-indol, farblose Kristalle, Schmp. 149–152 °C, $\alpha_D^{20}$: −54.9°, c = 1% (DMF).

7.25 1-Phenyl-3-[N-(toluol-4'-sulfonyl)-L-alanyloxyl]-indol

7.26. 1-Acetyl-3-[N-(toluol-4'-sulfonyl)-L-alanyloxy]-indol

7.27. 1-Benzoyl-3-[N-(toluol-4'-sulfonyl)-L-alanyloxy]-indol, farblose Kristalle, Schmp. 155–156 °C, $\alpha_D^{20}$: −54.7 °, c = 1% (DMF).

7.28. 3-[N-(Benzyloxycarbonyl)-glycyloxy]-indol, farblose Kristalle, Schmp. 122–124 °C.

7.29. 3-[N-(Toluol-4'-sulfonyl)-glycyloxy]-indol, farblose Kristalle, Schmp. 103–105 °C.

7.30. 3-[N-(Toluol-2'-sulfonyl)-L-alanyloxy]-indol farbloses amorphes Pulver; $\alpha_D^{20}$: −62.5°, c = 1% (Methanol) DC: Fertigplatte Kieselgel (Laufmittel: Toluol-Dioxan 9:1 Detektion: UV, $NH_3$ (Gas), $R_F$-Wert: 0,20)

7.31. 3-[N-(Toluol-3'-sulfonyl)-L-alanyloxy]-indol farblose Kristalle, Schmp. 94 °C; $\alpha_D^{20}$: −54.9°, c = 1% (Methanol)

7.32. 3-[N-(Toluol-4'-sulfonyl)-D-alanyloxy]-indol, farblose Kristalle, Schmp. 98 °C, $\alpha_D^{20}$: +53.1°, c = 1% (Methanol).

7.33. 3-[N-(Benzyloxycarbonyl)-D,L-alanyloxy]-indol, farblose Kristalle, Schmp. 110–111 °C.

7.34. 3-[N-(Benzyloxycarbonyl)-L-valyloxy]-indol, farblose Kristalle, Schmp. 64–65 °C, $\alpha_D^{20}$: −41.1°, c = 1% (Methanol).

7.35. 3-[N-(Benzyloxycarbonyl)-L-leucyloxy]-indol, farblose Kristalle, Schmp. 73–75 °C, $\alpha_D^{20}$: −42.2°, c = 1% (Methanol).

7.36. 3-[N-(Benzyloxycarbonyl)-L-isoleucyloxy]-indol, farbloses, amorphes Pulver, $\alpha_D^{20}$: −30.6°, c = 1% (Methanol), DC: Fertigplatte Kieselgel, (Laufmittel: Toluol-Dioxan 2:1, Detektion: UV, $NH_3$ (Gas), $R_F$-Wert: 0.57).

7.37. 3-[N-(Benzyloxycarbonyl)-L-phenylalanyloxy]-indol, gelbliche Kristalle, Schmp. 125 °C, DC: Fertigplatte Kieselgel, (Laufmittel: Toluol-Dioxan 9:1, Detektion: UV, $NH_3$ (Gas), $R_F$-Wert: 0.56).

7.38. 3-[N-(Toluol-4'-sulfonyl)-L-phenylalanyloxy]-indol, farblose Kristalle, Schmp. 167–169 °C, $\alpha_D^{20}$: −34.4°, c = 1% (Methanol).

7.39. 3-[N-Acetyl-L-tyrosyloxy]-indol

7.40. 3-[N-Benzoyl-L-tyrosyloxy]-indol

7.41. 3-[N-(Benzyloxycarbonyl)-L-tyrosyloxy]-indol, farbloser, amorpher Schaum, $\alpha_D^{20}$: −22.6°, c = 1% (Methanol), DC: Fertigplatte Kieselgel, (Laufmittel: Toluol-Dioxan 4:1, Detektion: UV, $NH_3$ (Gas), $R_F$-Wert: 0.27).

7.42. 3-[N-(Toluol-4'-sulfonyl)-L-tyrosyloxy]-indol, farblose Kristalle, Schmp. 171 °C, $\alpha_D^{20}$: −28.3°, c = 1% (Methanol).

7.43. 3-[N-(Toluol-4'-sulfonyl)- O-acetyl-L-tyrosyloxy]-indol, farblose Kristalle, Schmp. 168–170 °C, $\alpha_D^{20}$: −24.1°, c = 1% (Dimethylformamid).

7.44. 3-[N-(Benzyloxycarbonyl)-L-alanyl-L-alanyloxy]-indol, farblose Kristalle, Schmp. 144 °C, $\alpha_D^{20}$: −17.3°, c = 1% (Methanol).

7.45. 3-[N-(Toluol-4'-sulfonyl)-D-alanyl-L-alanyloxy]-indol, gelblicher, amorpher Schaum, $\alpha_D^{20}$: +5.8°, c = 1% (Methanol), DC: Fertigplatte Kieselgel, (Laufmittel: Toluol-Dioxan 4:1, Detektion: UV, $NH_3$ (Gas), $R_F$-Wert: 0.19).

7.46. 3-[N-(Benzyloxycarbonyl)-L-alanyl-L-alanyl-L-alanyloxy]-indol, farblose Kristalle, Schmp. 156 °C, $\alpha_D^{20}$: −39.5°, c = 1% (Methanol).

7.47. 3-[N-(Toluol-4'-sulfonyl)-D-alanyl-D-alanyl-L-alanyloxy]-indol, farblose Kristalle, Schmp. 216 °C, $\alpha_D^{20}$: +53.8°, c = 1% (Methanol).

7.48. 3-[N-Acetyl-L-alanyloxy]-indol, farbloser, amorpher Schaum, $\alpha_D^{20}$: −12.5°, c = 1% (Methanol), DC: Fertigplatte Kieselgel, (Laufmittel: Essigester-Dichlormethan 10:1, Detektion: UV, $NH_3$ (Gas), $R_F$-Wert: 0.33).

7.49. 3-[N-Succinyl-L-alanyloxy]-indol, farblose Kristalle, Schmp. 142 °C, $\alpha_D^{20}$: −63.9°, c = 1% (Methanol).

7.50. 3-[N-Benzoyl-D,L-alanyloxy]-indol, farblose Kristalle, Schmp. 171 °C.

7.51. 3-[N-Phthaloyl-L-alanyloxy]-indol, farblose Kristalle, Schmp. 58 °C, $\alpha_D^{20}$: −26.3°, c = 1% (Methanol).

7.52. 3-[N-(Ethoxycarbonyl)-L-alanyloxy]-indol, farblose Kristalle, Schmp. 96 °C, $\alpha_D^{20}$: −68.9°, c = 1% (Methanol).

7.53. 3-[N-(Cyclohexyloxycarbonyl)-L-alanyloxy]-indol, farblose Kristalle, Schmp. 149 °C, $\alpha_D^{20}$: −60.4°, c = 1% (Methanol).

7.54. 3-[N-(Phenyloxycarbonyl)-L-alanyloxy]-indol, farblose Kristalle, Schmp. 161 °C, $\alpha_D^{20}$: −96.0°, c = 1% (Methanol).

7.55. 3-[N-(4'-Methyl-benzyloxycarbonyl)-L-alanyloxy]-indol, farblose Kristalle, Schmp. 135 °C, $\alpha_D^{20}$: −45.9°, c = 1% (Methanol).

7.56. 3-[N-(4'-Nitro-benzyloxycarbonyl)-L-alanyloxy]-indol, gelbliche Kristalle, Schmp. 160 °C, $\alpha_D^{20}$: −29.4°, c = 1% (Methanol).

7.57. 3-[N-(Benzylthiocarbonyl)-L-alanyloxy]-indol, farblose Kristalle, Schmp. 122 °C, $\alpha_D^{20}$: −77.8°, c = 1% (Methanol).

7.58. 3-[N-(Methansulfonyl)-L-alanyloxy]-indol, farblose Kristalle, Schmp. 164–166 °C, $\alpha_D^{20}$: −54.0°, c = 1% (Methanol).

7.59. 3-[N-(Benzylsulfonyl)-L-alanyloxy]-indol, farbloses, viskoses Öl, $\alpha_D^{20}$: −54.8°, c = 1% (Methanol), DC: Fertigplatte Kieselgel, (Laufmittel: Toluol-Essigester 2:1, Detektion: UV, $NH_3$ (Gas), $R_F$-Wert: 0.40).

7.60. 3-[N-(Benzolsulfonyl)-L-alanyloxy]-indol, farbloses, viskoses Öl, $\alpha_D^{20}$: −59.8°, c = 1% (Methanol), DC: Fertigplatte Kieselgel, (Laufmittel: Toluol-Essigester 2:1, Detektion: UV, $NH_3$ (Gas), $R_F$-Wert: 0.48).

7.61. 3[N-(4'-Brom-benzolsulfonyl)-L-alanyloxy]-indol, gelbliches, amorphes Pulver, DC: Fertigplatte Kieselgel, (Laufmittel: Toluol-Dioxan 4:1,

Detektion: UV, $NH_3$ (Gas), $R_F$-Wert: 0.39).

7.62. 3-[N-(4'-Nitro-benzolsulfonyl)-L-alanyloxy]-indol, farblose Kristalle, Schmp. 136–137 °C, $\alpha_D^{20}$: −35.9°, c = 1% (Methanol).

7.63. 3-[N-(4'-Acetylamino-benzolsulfonyl)-L-alanyloxy]-indol, farblose Kristalle, Schmp. 162–163 °C, $\alpha_D^{20}$: −57.2°, c = 1% (Methanol).

7.64. 3-[N-(4'-n-Butyl-benzolsulfonyl)-L-alanyloxy]-indol, farblose Kristalle, Schmp. 105 °C, $\alpha_D^{20}$: −46.1°, c = 1% (Methanol).

7.65. 3-[N-(4'-tert.-Butyl-benzolsulfonyl)-L-alanyloxy]-indol, farblose Kristalle, Schmp. 140 °C, $\alpha_D^{20}$: −43.3°, c = 1% (Methanol).

7.66. 3-[N-(4'-n-Octyl-benzolsulfonyl)-L-alanyloxy]-indol, farblose Kristalle, Schmp. 57 °C, $\alpha_D^{20}$: −34.2°, c = 1% (Methanol).

7.67. 3-[N-(4'-Hydroxy-benzolsulfonyl)-L-alanyloxy]-indol, viskoses, schwach rötliches Öl, DC: Fertigplatte Kieselgel, (Laufmittel: Toluol-Dioxan 1:1, Detektion: UV, $NH_3$ (Gas), $R_F$-Wert: 0.62).

7.68. 3-[N-(4'-Methoxy-benzolsulfonyl)-L-alanyloxy]-indol, farblose Kristalle, Schmp. 126–128 °C, $\alpha_D^{20}$: −54.3°, c = 1% (Methanol).

7.69. 3-[N-(4'-Benzyloxy-benzolsulfonyl)-L-alanyloxy]-indol, farblose Kristalle, Schmp. 112 °C, $\alpha_D^{20}$: −40.3°, c = 1% (Methanol).

7.70. 3-[N-(4'-⟨2''-Hydroxy-ethoxy⟩-benzolsulfonyl)-L-alanyloxy]-indol

7.71. 3-[N-(4'-⟨3''-Oxa-5''-hydroxy-n-pentyloxy⟩-benzolsulfonyl)-L-alanyloxy]-indol farbloses, viskoses Öl, $\alpha_D^{20}$: −36.4°, c = 1% (Methanol) DC: Fertigplatte Kieselgel (Laufmittel: Toluol-Essigsäureethylester 1:10, Detektion: UV, $NH_3$ (Gas), $R_F$-Wert: 0.37

7.72. 3-[N-(4'-⟨3'',6''-Di-oxa-n-heptyloxy⟩-benzolsulfonyl)-L-alanyloxy]-indol, schwach rötliches, viskoses Öl, DC: Fertigplatte Kieselgel, (Laufmittel: Toluol-Dioxan 4:1, Detektion: UV, $NH_3$ (Gas), $R_F$-Wert: 0.21).

7.73. 3-[N-(4'-⟨2''-Hydroxy-ethyl⟩-benzolsulfonyl)-L-alanyloxy]-indol, gelbliches, amorphes Pulver, $\alpha_D^{20}$: −54.5°, c = 1% (Methanol) DC: Fertigplatte, Kieselgel (Laufmittel: Toluol-Essigsäureethylester 1:2, Detektion: UV, $NH_3$ (Gas), $R_F$-Wert: 0.26).

7.74. 3-[N-(4'-⟨2''-{4'''-Nitro-benzyloxy}-ethyl⟩-benzolsulfonyl)-L-alanyloxy]-indol

7.75. 3-[N-(4'-⟨2''-Chlor-ethyl⟩-benzolsulfonyl)-L-alanyloxy]-indol, schwach rötlicher, amorpher Schaum, $\alpha_D^{20}$: −48.3°, c = 1% (Methanol), DC: Fertigplatte Kieselgel, (Laufmittel: Toluol-Essigester 5:1, Detektion: UV, $NH_3$ (Gas), $R_F$-Wert: 0.20).

7.76. 3-[N-(4'-Cyano-benzolsulfonyl)-L-alanyloxy]-indol farblose Kristalle, Schmp. 131 °C; $\alpha_D^{20}$: −47.0°, c = 1% (Dimethylformamid).

7.77. 3-[N-(4'-Carboxy-benzolsulfonyl)-L-alanyloxy]-indol, farblose Kristalle, Schmp. 143 °C, $\alpha_D^{20}$: −51.0°, c = 1% (Methanol).

7.78. 3-[N-(4'-Benzyloxycarbonyl-benzolsulfonyl)-L-alanyloxy]-indol, farblose Kristalle, Schmp. 178 °C, $\alpha_D^{20}$: −42.8°, c = 1% (Aceton).

7.79. 3-[N-(4'-Carbamoyl-benzolsulfonyl)-L-alanyloxy]-indol, farblose Kristalle, Schmp. 206 °C, $\alpha_D^{20}$: −58.0°, c = 1% (Aceton).

7.80. 3-[N-(4'-⟨Dimethyl-carbamoyl⟩-benzolsulfonyl)-L-alanyloxy]-indol, farblose Kristalle, Schmp. 170–172 °C, $\alpha_D^{20}$: −46.4°, c = 1% (Dimethylformamid).

7.81. 3-[N-Methyl-N-(toluol-4'-sulfonyl)-L-alanyloxy]-indol, farblose Kristalle, Schmp. 82 °C, $\alpha_D^{20}$: −42.2°, c = 1% (Methanol).

7.82. 3-[N-(2',4',6'-Trimethyl-benzolsulfonyl)-L-alanyloxy]-indol, farblose Kristalle, Schmp. 192–194 °C, $\alpha_D^{20}$: −63.8°, c = 1% (Methanol).

7.83. 3-[N-(Biphenyl-4'-sulfonyl)-L-alanyloxy]-indol, farblose Kristalle, Schmp. 159 °C, $\alpha_D^{20}$: −42.0°, c = 1% (Methanol).

7.84. 3-[N-(Naphthalin-2'-sulfonyl)-L-alanyloxy]-indol, schwach rötliche Kristalle, Schmp. 103–105 °C, DC: Fertigplatte Kieselgel, (Laufmittel: Toluol-Dioxan 4:1, Detektion: UV, $NH_3$ (Gas), $R_F$-Wert: 0.41).

7.85. 3-[N-(4'-Acetylamino-naphthalin-1'-sulfonyl)-L-alanyloxy]-indol, farblose Kristalle, Schmp. 129–132 °C, $\alpha_D^{20}$: −63.2°, c = 1% (Methanol).

7.86. 3-[N-(5'-Dimethylamino-naphthalin-1'-sulfonyl)-L-alanyloxy]-indol, farblose Kristalle, Schmp. 170 °C, $\alpha_D^{20}$: −30.7°, c = 1% (Methanol).

7.87. 3-[N-(Benzyloxycarbonyl)-L-alanyloxy]-benzo-[b]-thiophen, farbloses, viskoses Öl, $\alpha_D^{20}$: −42.2°, c = 1% (Methanol), DC: Fertigplatte Kieselgel, (Laufmittel: Dichlormethan, Detektion: UV, NaOH / $K_3Fe(CN)_6$, $R_F$-Wert: 0.13).

7.88. 3-[N-(Toluol-4'-sulfonyl)-L-alanyloxy]-5-benzyl-indol

7.89. 3-[N-(Toluol-4'-sulfonyl)-L-alanyloxy]-5-phenyl-indol

Beispiel 8

3-[N-(tert.-Butyloxycarbonyl)-L-alanyloxy]-indol

Zur Umsetzung nach dem Carbodimid-Verfahren werden 3.23 g (0.017 mol) N-tert.-Butyloxycarbonyl-L-alanin und 2.27 g (0.017 mol) Indoxyl in 100 ml abs. Dioxan-Dichlormethan 1:1 gelöst. Nach Zugabe einer Lösung von 3.87 g (0.019 mol) Dicyclohexylcarbodiimid (DCC) in 20 ml abs. Dioxan rührt man unter Wasser- und Sauerstoffausschluss 68 h bei Raumtemperatur. Der abgeschiedene N,N'-Dicyclohexylharnstoff wird abgesaugt, das Lösungsmittel im Vakuum abdestilliert und der Rückstand in ca. 100 ml Essigsäureethylester aufgenommen. Nach Absaugen von weiterem ausgefallenem N,N'-Dicyclohexylharnstoff wird die klare Essigesterlösung nacheinander je zweimal mit 30 ml 1 N Zitronensäure, 20 ml Wasser, 50 ml 5–10-proz. Natriumhydrogencarbonatlösung und 25 ml Wasser gewaschen. Nach Trocknen über Natriumsulfat wird die Essigesterphase im Vakuum eingeengt. Der amorphe Rückstand wird säulenchromatographisch an Kieselgel mit einem Toluol-Dioxan-Gemisch (9:1) gereinigt.

Nach Einengen der gesammelten Fraktionen im Vakuum und Umkristallisieren des Rückstandes aus Essigester-Ether (1:10) erhält man
1.04 g (20%) 3-[N-(tert. -Butyloxycarbonyl)-L-alanyloxy]-indol,
farblose Kristalle, Schmp. 158 °C,
$\alpha_D^{20}$: −65.0°, c = 1% (Methanol).

Als Nebenprodukt entsteht auch bei dieser Umsetzung das in 1-Stellung substituierte 3-Hydroxy-indol (s. Beispiel 7).

In analoger Weise erhält man durch Umsetzung der entsprechend substituierten Indoxyl-Verbindungen mit den jeweiligen Aminosäuren die folgenden Substrate, wobei auch hier in allen Fällen als Nebenprodukte die entsprechenden in 1-Stellung substituierten 3-Hydroxy-indole entstehen:

8.1. 3-[N-Formyl-L-alanyloxy]-indol,
farblose Kristalle, Schmp. 121–122 °C,
$\alpha_D^{20}$: −2.5°, c = 1% (Methanol).

8.2. 3-[N-(4 '-Methoxy-benzyloxycarbonyl)-L-alanyloxy]-indol,
farblose Kristalle, Schmp. 92 °C,
$\alpha_D^{20}$: −42.9°, c = 1% (Methanol).

8.3. 3-[N-(N-⟨Piperidino⟩-oxycarbonyl)-L-alanyloxy]-indol,
farblose Kristalle, Schmp. 104 °C,
$\alpha_D^{20}$: −24.7°, c = 1% (Methanol).

8.4. 3-[N-(Thienyl-(2')-methoxycarbonyl)-L-alanyloxy]-indol,
farblose Kristalle, Schmp. 134 °C,
$\alpha_D^{20}$: −55.7°, c = 1% (Methanol).

8.5. 3-[N-(Chinolin-8'-sulfonyl)-L-alanyloxy]-indol,
amorphes, schwach rötliches Pulver,
DC: Fertigplatte Kieselgel,
(Laufmittel: Toluol-Dioxan 4:1,
Detektion: UV, $NH_3$ (Gas),
$R_F$-Wert: 0.23).

8.6. 3-[N-(Diphenylcarbamoyl)-L-alanyloxy]-indol,
farblose Kristalle, Schmp. 188–190 °C,
$\alpha_D^{20}$: +22.2°, c = 1% (Pyridin).

Beispiel 9

3-[N-(Furyl-⟨2'⟩-methoxycarbonyl)-L-alanyloxy]-indol

Zur Umsetzung nach dem Aktivester-Verfahren werden 4.26 g (0.02 mol) N-(Furyl-⟨2'⟩-methoxycarbonyl)-L-alanin und 5.4 g (0.04 mol) N-Hydroxy-benzotriazol in 50 ml abs. Tetrahydrofuran (THF) gelöst, auf 0 °C abgekühlt und mit einer Lösung von 4.4 g (0.022 mol) Dicyclohexylcarbodiimid (DCC) in 10 ml abs. THF versetzt. Zur Bildung des Aktivesters rührt man das Reaktionsgemisch 1.5 h bei 0 °C, dann 2 h bei Raumtemperatur. Darauf fügt man unter Sauerstoff- und Wasserausschluss 2.66 g (0.02 mol) Indoxyl und 2.77 ml (0.04 mol) abs. Triethylamin zu. Das Gemisch wird 18 h bei Raumtemperatur gerührt. Der gebildete N,N'-Dicyclohexylharnstoff wird abgesaugt, das Lösungsmittel wird im Vakuum abdestilliert und der Rückstand wird wie in Beispiel 7 beschrieben aufgearbeitet. Das amorphe Rohprodukt wird dann säulenchromatographisch an einer Kieselgelsäule mit einem Toluol-Essigester-Gemisch (5:1) gereinigt. Nach Einengen der entsprechenden gesammelten Fraktionen und Umkristallisation aus Essigester-Petrolether 1:1 ergeben sich
3.0 g (45.7%) 3-[N-(Furyl-⟨2'⟩-methoxycarbonyl)-L-alanyloxy]-indol,
farblose Kristalle, Schmp. 129 °C,
$\alpha_D^{20}$: −51.9°, c = 1% (Methanol).

In analoger Weise erhält man durch Reaktion der entsprechend substituierten Aminosäuren oder Peptide mit den jeweiligen Indoxyl-Verbindungen die folgenden Substanzen:

9.1. 3-[N-(Toluol-4'-sulfonyl-L-valyloxy]-indol,
farblose Kristalle, Schmp. 125–127 °C,
$\alpha_D^{20}$: −24.5°, c = 1% (Methanol).

9.2. 3-[N-(4'-Dimethylamino-benzolsulfonyl)-L-alanyloxy]-indol,
amorphes, schwach rötliches Pulver,
DC: Fertigplatte Kieselgel,
(Laufmittel: Toluol-Dioxan 3:1,
Detektion: UV, $NH_3$ (Gas),
$R_F$-Wert: 0.25).

9. 3. 3-[N-(4'-Acetyl-benzolsulfonyl)-L-alanyloxy]-indol

9.4. 3-[N-(4'-Methoxycarbonyl-benzolsulfonyl)-L-alanyloxy]-indol,
farblose Kristalle, Schmp. 140–141 °C,
$\alpha_D^{20}$: −40.3°, c = 1% (Methanol).

9.5. 3-[N-(4'-Carboxymethyl-benzolsulfonyl)-L-alanyloxy]-indol, amorpher, farbloser Schaum, $\alpha_D^{20}$: −82.1°, c = 1% (Methanol), DC: Fertigplatte Kieselgel, (Laufmittel: Isopropanol-Essigsäure-n-butylester-Wasser 5:3:2, Detektion: UV, $NH_3$ (Gas), $R_F$-Wert: 0.68).

9.6. 3-[N-(4'Carboxymethoxy-benzolsulfonyl)-L-alanyloxy]-indol, amorpher, farbloser Schaum, $\alpha_D^{20}$: −47.7°, c = 1% (Methanol), DC: Fertigplatte Kieselgel, (Laufmittel: Toluol-Dioxan-Eisessig 6:2:1, Detektion: UV, $NH_3$ (Gas), $R_F$-Wert: 0.27).

9.7. 3-[N-(4'-Carboxymethylamino-benzolsulfonyl)-L-alanyloxy]-indol, farblose Kristalle, Schmp. 151–153 °C $\alpha_D^{20}$: −71.5°, c = 1% (Methanol).

9.8. 3-[N'-(4'-⟨Benzyloxycarbonyl-methylamino⟩-benzolsulfonyl)-L-alanyloxy]-indol, farblose Kristalle, Schmp. 110–113 °C, $\alpha_D^{20}$: −60.0°, c = 1% (Methanol).

9.9. 3-[N-(4'-Fluor-benzolsulfonyl)-L-alanyloxy]-indol, farblose Kristalle, Schmp. 94–95 °C, $\alpha_D^{20}$: −48.5°, c = 1% (Methanol).

9.10. 3-[N-(4'-Fluorsulfonyl-benzolsulfonyl)-L-alanyloxy]-indol

9.11. 3-[N-(4'-Sulfamoyl-benzolsulfonyl)-L-alanyloxy]-indol

9.12. 3-[N-Acetyl-N-(toluol-4'-sulfonyl)-L-alanyloxy]-indol, farbloses, amorphes Pulver, $\alpha_D^{20}$: −10.6°, c = 1% (Methanol), DC: Fertigplatte Kieselgel, (Laufmittel: Toluol-Dioxan 9:1, Detektion: UV, $NH_3$ (Gas), $R_F$-Wert: 0.17).

9.13. 3-[N-(Pyridin-3'- sulfonyl)-L-alanyloxy]-indol, farblose Kristalle, Schmp. 155–157 °C, $\alpha_D^{20}$: −55.1°, c = 1% (Methanol).

9.14. 3-[N-(5',5'-Dimethyl-3'-oxo-cyclohexen-1'-yl)-L-alanyloxy]-indol. farblose Kristalle, Schmp. 154 °C, $\alpha_D^{20}$: −272.2°, c = 1% (Methanol).

9.15. 3-[N-(Di-⟨4'-nitrobenzyl⟩-phosphoryl)-L-alanyloxy]-indol

9.16. 3-[N-(Di-⟨4'-brombenzyl⟩-phosphoryl)-L-alanyloxy]-indol

9.17. 3-[N-(Toluol-4'-sulfonyl)-L-alanyloxy]-5-methoxy-indol farbloses, viskoses Öl; $\alpha_D^{20}$: −46.8°, c = 1% (Methanol), DC:Fertigplatte, Kieselgel (Laufmittel: Toluol-Essigsäureethylester 2:1 Detektion: UV, $NH_3$ (Gas), $R_F$-Wert: 0.45).

---

Beispiel 10

3-[ N-(3',6'-Dioxa-n-heptyloxy-carbonyl)-L-alanyl-oxy]-indol

Zur Umsetzung nach dem Mischanhydrid-Verfahren werden 3.52 g (0.015 mol) N-(3,6-Dioxa-n-heptyloxy-carbonyl)-L-alanin in 35 ml abs. Tetrahydrofuran (THF) gelöst. Nach Zugabe von 2.1 ml (0.015 mol) Triethylamin wird das Gemisch auf −15 °C abgekühlt und dann zur Bildung des Mischanhydrids mit 1.43 ml (0.015 mol) Chlorameisensäureethylester versetzt. Nach 1 h Rühren bei −15 °C fügt man unter Sauerstoff- und Wasserausschluss eine auf −15 °C gekühlte Lösung von 2.00 g (0.015 mol) Indoxyl in 20 ml abs. THF zu. Das Gemisch wird weitere 2 h bei −15 °C bis −20 °C gerührt und dann über Nacht im Kühlschrank belassen. Das gebildete Triethylaminhydrochlorid wird abgesaugt, das Lösungsmittel wird im Vakuum abgezogen und der Rückstand wird wie in Beispiel 7 beschrieben aufgearbeitet. Das amorphe Rohprodukt wird dann säulenchromatographisch an Kieselgel zunächst mit einem Methylenchlorid-Methanol-Gemisch (95:5) und dann mit einem Toluol-Methylethylketon-Gemisch (1:2) gereinigt. Nach Behandeln der gesammelten Fraktionen mit Aktivkohle und Abziehen des Lösungsmittels ergeben sich

0.51 g (9.5%) 3-[N-(3',6'-Dioxa-n-heptyloxy-carbonyl)-L-alanyloxy]-indol, farbloses, viskoses Öl, $\alpha_D^{20}$: −40.4°, c = 1% (Methanol), DC: Fertigplatte Kieselgel, (Laufmittel: Toluol-Methylethylketon 1:2, Detektion: UV, $NH_3$ (Gas), $R_F$-Wert: 0.54).

In analoger Weise erhält man durch Reaktion der entsprechend substituierten Aminosäuren mit den jeweiligen Indoxyl-Verbindungen die folgenden Substanzen:

---

10.1. 3-[N-(2'-Nitro-benzolsulfenyl)-L-alanyloxy]-indol, gelbe Kristalle, Schmp. 133–134 °C, $\alpha_D^{20}$: −116.0°, c = 1% (Methanol).

10.2. 3-[N-(1'-Methyl-2'-benzoyl-vinyl)-L-alanyloxy]-indol, schwach rötliche Kristalle, Schmp. 130–133 °C, DC: Fertigplatte Kieselgel, (Laufmittel: Toluol-Dioxan 4:1, Detektion: UV, $NH_3$ (Gas), $R_F$-Wert: 0.45).

---

**Patentansprüche**

1. Diagnostisches Mittel zum Nachweis proteolytischer Enzyme, bestehend aus einem saugfähigen Träger, einer Filmschicht, einer Pulvermischung, einem Lyophilisat, einer Lösung oder einer Reagenztablette, enthaltend ein oder mehrere Chromogene und eine geeignete Puffersubstanz, dadurch gekennzeichnet, dass als Chromogene Indoxyl- und/oder Thioindoxyl-Aminosäureester und/oder -Peptidester der allgemeinen Formel I

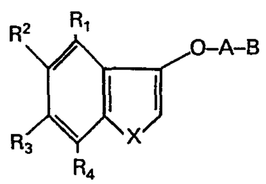

(I),

in der
$R_1$, $R_2$, $R_3$, $R_4$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,
X ein Schwefelatom oder eine gegebenenfalls durch einen niederen Alkyl-, einen Aryl-, einen Aralkyl- oder einen Acylrest substituierte Iminogruppe,
A einen Aminosäure- oder einen Peptidrest,
B eine in der Peptidchemie übliche oder davon abgeleitete Stickstoffschutzgruppe bedeutet,
eingesetzt werden.

2. Diagnostisches Mittel gemäss Anspruch 1, dadurch gekennzeichnet, dass zusätzliche üblicherweise verwendete Hilfsstoffe zugesetzt werden.

3. Diagnostisches Mittel gemäss Anspruch 2, dadurch gekennzeichnet, dass als zusätzliche Hilfsmittel Netzmittel, Oxidationsmittel, Filmbildner, galenische Zusatzstoffe und/oder Gerüstbildner verwendet werden.

4. Verwendung von Indoxyl- und/oder Thioindoxyl-Aminosäureestern und/oder -Peptidestern der allgemeinen Formel I

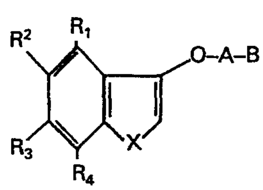

(I),

in der
$R_1$, $R_2$, $R_3$, $R_4$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,
X ein Schwefelatom oder eine gegebenenfalls durch einen niederen Alkyl-, einen Aryl-, einen Aralkyl- oder einen Acylrest substituierte Iminogruppe,
A einen Aminosäure- oder einen Peptidrest,
B eine in der Peptidchemie übliche davon abgeleitete Stickstoffschutzgruppe bedeutet,
zur Herstellung diagnostischer Mittel zum Nachweis proteolytischer Enzyme.

5. Indoxyl- und Thioindoxyl-Aminosäureester und -Peptidester der allgemeinen Formel I

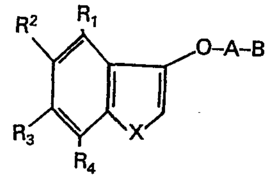

(I),

in der
$R_1$, $R_2$, $R_3$, $R_4$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,
X ein Schwefelatom oder eine gegebenenfalls durch einen niederen Alkyl-, einen Aryl-, einen Aralkyl- oder einen Acylrest substituierte Iminogruppe,
A einen Aminosäure- oder einen Peptidrest,
B eine in der Peptidchemie übliche oder davon abgeleitete Stickstoffschutzgruppe bedeutet.

6. Verfahren zur Herstellung von Indoxyl- und Thioindoxyl-Aminosäureestern und -Peptidestern der allgemeinen Formel I

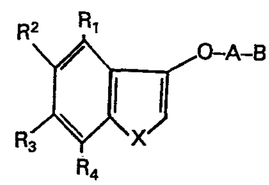

(I),

in der
$R_1$, $R_2$, $R_3$, $R_4$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,
X ein Schwefelatom oder eine gegebenenfalls durch einen niederen Alkyl-, einen Aryl-, einen Aralkyl- oder einen Acylrest substituierte Iminogruppe,
A einen Aminosäure- oder einen Peptidrest,
B eine in der Peptidchemie übliche oder davon abgeleitete Stickstoffschutzgruppe bedeutet,
dadurch gekennzeichnet, dass man in an sich bekannter Weise Verbindungen der allgemeinen Formel II

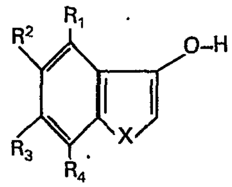

(II),

in der
$R_1$, $R_2$, $R_3$, $R_4$ und X die oben angegebene Bedeutung haben, mit Aminosäuren und Peptiden der allgemeinen Formel III

H–O–A–B (III),

in der
A und B die oben angegebene Bedeutung haben, beziehungsweise mit reaktiven Derivaten davon, umsetzt.

**Revendications**

1. Agent de diagnostic pour le décèlement de germes protéolytiques, comprenant un support absorbant, une couche de film, un mélange pulvérulent, un lyophilisat, une solution ou un comprimé contenant les réactifs, renfermant un ou plusieurs agents chromogènes et une substance tampon appropriée, caractérisé en ce que les agents chromogènes sont des esters d'indoxyle et/ou de thio-indoxyle avec des amino-acides et/ou avec des peptides, de formule générale I:

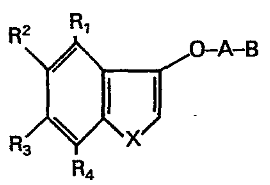

(I),

dans laquelle:
$R_1$, $R_2$, $R_3$, $R_4$, qui peuvent être identiques ou différents, sont chacun un atome d'hydrogène ou d'halogène, un groupe alkyle inférieur, un groupe alcoxy, aryle, aralkyle ou aralcoxy inférieur, un groupe hydroxyle, un groupe carboxyle, un groupe carboxy-alcoxy inférieur, un groupe aralcoxy-carbonyle, un groupe aralcoxycarbonyl-alcoxy inférieur, un groupe nitro ou un groupe acylamino inférieur, ou bien chaque fois deux substituants voisins forment un reste de structure benzénique éventuellement substitué par de l'halogène,
X est un atome de soufre ou un groupe imino substitué éventuellement par un reste alkyle inférieur, aryle, aralkyle ou acyle,
A est un reste d'un amino-acide ou d'un peptide,
B est un groupe de protection de l'azote habituellement utilisé dans la chimie des peptides ou un dérivé de ce groupe.

2. Agent de diagnostic selon la revendication 1, caractérisé en ce que l'on ajoute des additifs complémentaires habituellement utilisés.

3. Agent de diagnostic selon la revendication 2, caractérisé en ce que l'on emploie comme additifs complémentaires des agents mouillants, des agents oxydants, des agents filmogènes, des additifs galéniques et/ou des agents consolidants.

4. Utilisation d'esters d'indoxyle et/ou de thio-indoxyle avec des amino-acides et/ou avec des peptides, de formule générale I:

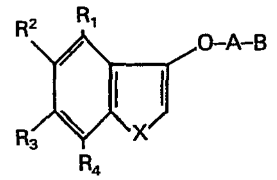

(I),

dans laquelle:
$R_1$, $R_2$, $R_3$, $R_4$ qui peuvent être identiques ou différents, sont chacun un atome d'hydrogène ou d'halogène, un groupe alkyle inférieur, un groupe alcoxy, aryle, aralkyle ou aralcoxy, un groupe hydroxyle, un groupe carboxyle, un groupe carboxy-alcoxy inférieur, un groupe aralcoxy-carbonyle, un groupe aralcoxycarbonyl-alcoxy inférieur, un groupe nitro ou un groupe acylamino inférieur, ou bien chaque fois deux substituants voisins forment un reste de structure benzénique, éventuellement substitué par de l'halogène,
X est un atome de soufre ou un groupe imino substitué éventuellement par un reste alkyle inférieur aryle, aralkyle ou acyle,
A est un reste d'un amino-acide ou d'un peptide,
B est un groupe de protection de l'azote habituellement utilisé dans la chimie des peptides ou un dérivé de ce groupe,
pour la fabrication d'agents de diagnostic pour le décèlement d'enzymes protéolytiques.

5. Esters d'indoxyle et de thio-indoxyle avec des amino-acides et/ou avec des peptides, de formule générale I

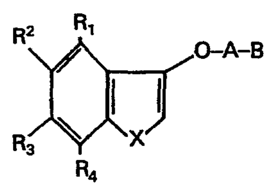

(I),

dans laquelle:
$R_1$, $R_2$, $R_3$, $R_4$, qui peuvent être identiques ou différents, sont chacun un atome d'hydrogène ou d'halogène, un groupe alkyle inférieur , un groupe alcoxy, aryle, aralkyle ou aralcoxy inférieur, un groupe hydroxyle, un groupe carboxyle, un groupe carboxy-alcoxy inférieur, un groupe aralcoxy-carbonyle, un groupe aralcoxycarbonyl-alcoxy inférieur, un groupe nitro ou un groupe acylamino inférieur, ou bien chaque fois deux substituants voisins forment un reste de structure benzénique éventuellement substitué par de l'halogène,
X est un atome de soufre ou un groupe amino substitué éventuellement par un reste alkyle inférieur, aryle, aralkyle ou acyle,
A est un reste d'un amino-acide ou d'un peptide,
B est un groupe de protection de l'azote habituellement utilisé dans la chimie des peptides ou un dérivé de ce groupe.

6. Procédé de préparation des esters d'indoxyle et de thio-indoxyle avec des amino-acides et/ou avec des peptides, de formule générale I

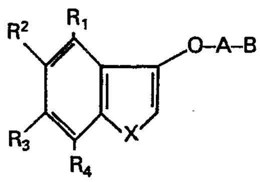

(I),

dans laquelle:
$R_1$, $R_2$, $R_3$ et $R_4$, qui peuvent être identiques ou différents, sont chacun un atome d'hydrogène ou d'halogène, un groupe alkyle inférieur, un groupe alcoxy, aryle, aralkyle ou aralcoxy inférieur, un groupe hydroxyle, un groupe carboxyle, un groupe carboxy-alcoxy inférieur, un groupe aralcoxy-carbonyle, un groupe aralcoxycarbonyl-alcoxy inférieur, un groupe nitro ou un groupe acylamino inférieur, ou bien chaque fois deux substituants voisins forment un reste de structure benzénique éventuellement substitué par de l'halogène,
X est un atome de soufre ou un groupe amino substitué éventuellement par un reste alkyle inférieur, aryle, aralkyle ou acyle,
A est un reste d'un amino-acide ou d'un peptide,
B est un groupe de protection de l'azote habituellement utilisé dans la chimie des peptides ou un dérivé de ce groupe,
caractérisé en ce que l'on fait réagir de façon en soi connue des composés de formule générale II

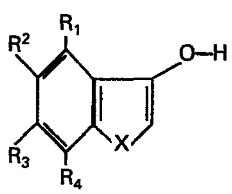

(II),

dans laquelle:
$R_1$, $R_2$, $R_3$, $R_4$ et X ont la signification indiquée ci-dessus, avec des amino-acides et des peptides de formule générale III

H–O–A–B (III),

dans laquelle:
A et B ont la signification ci-dessus indiquée, ou bien avec des dérivés réactifs de ces composés.

**Claims**

1. Diagnostic agent for the detection of proteolytic enzymes, consisting of an absorbent carrier, a film layer, a powder mixture, a lyophilisate, a solution or a reagent tablet, containing one or more chromogens and a suitable buffer substance, characterised in that as chromogens there are used indoxyl- and/or thioindoxylamino acid esters and/or peptide esters of the general formula I

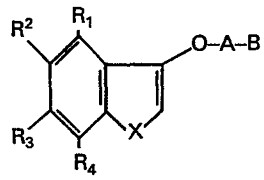

(I),

in which
$R_1$, $R_2$, $R_3$, $R_4$, which can be the same or different, each signify hydrogen or halogen, a lower alkyl, lower alkoxy, aryl, aralkyl, aralkoxy, a hydroxyl, a carboxy, a carboxy lower alkoxy, an aralkoxycarbonyl, an aralkyloxycarbonyl lower alkoxy, a nitro or a lower acylamino group or two neighbouring substituents a benzo-annellated radical possibly substituted by halogen,
X a sulphur atom or an imino group possibly substituted by a lower alkyl, an aryl, an aralkyl or an acyl radical,
A an amino acid or a peptide residue,
B a nitrogen protective group conventional in peptide chemistry or derived therefrom.

2. Diagnostic agent according to claim 1, characterised in that conventionally used adjuvants are additionally added.

3. Diagnostic agent according to claim 2, characterised in that wetting agents, oxidation agents, film formers, galenical additives and/or structural formers are employed as additional ajuvants.

4. Use of indoxyl- and/or thioindoxyl-amino acid esters and/or peptide esters of the general formula I

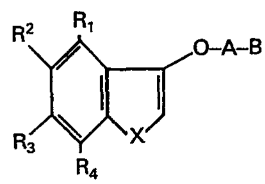

(I),

in which
$R_1$, $R_2$, $R_3$, $R_4$, which can be the same or different, each signify hydrogen or halogen, a lower alkyl, lower alkoxy, aryl, aralkyl, aralkoxy, a hydroxyl, a carboxy, a carboxy lower alkoxy, an aralkoxycarbonyl, an aralkyloxycarbonyl lower alkoxy, a nitro or a lower acylamino group of two neighbouring substituents a benzo-annellated radical possibly substituted by halogen,
X a sulphur atom or an imino group possibly substituted by a lower alkyl, an aryl, an aralkyl or an acyl radical,
A an amino acid or a peptide residue,
B a nitrogen protective group conventional in peptide chemistry or derived therefrom, for
the production of diagnostic agents for the detection of proteolytic enzymes.

5. Indoxyl and thioindoxyl-amino acid esters and peptide esters of the general formula I

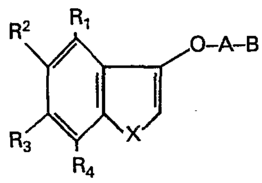

(I),

in which
$R_1$, $R_2$, $R_3$, $R_4$, which can be the same or different, each signify hydrogen or halogen, a lower alkyl, lower alkoxy, aryl, aralkyl, aralkoxy, a hydroxyl, a carboxy, a carboxy lower alkoxy, an aralkoxycarbonyl, an aralkyloxycarbonyl lower alkoxy, a nitro or a lower acylamino group or two neighbouring substituents a benzo-annellated radical possibly substituted by halogen,
X a sulphur atom or an imino group possibly substituted by a lower alkyl, an aryl, an aralkyl or an acyl radical,
A an amino acid or a peptide residue,
B a nitrogen protective group conventional in peptide chemistry or derived therefrom.

6. Process for the preparation of indoxyl- and thioindoxyl-amino acid esters and peptide esters of the general formula I

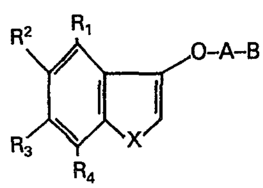

(I),

in which
$R_1$, $R_2$, $R_3$, $R_4$, which can be the same or different, each signify hydrogen or halogen, a lower alkyl, lower alkoxy, aryl, aralkyl, aralkoxy, a hydroxyl, a carboxy, a carboxy lower alkoxy, an aralkoxycarbonyl, an aralkyloxycarbonyl lower alkoxy, a nitro or a lower acylamino group or two neighbouring substituents a benzo-annellated radical possibly substituted by halogen,
X a sulphur atom or an imino group possibly substituted by a lower alkyl, an aryl, an aralkyl or an acyl radical,
A an amino acid or a peptide residue,
B a nitrogen protective group conventional in peptide chemistry or derived therefrom,
characterised in that, in per se known manner, one reacts compounds of the general formula II

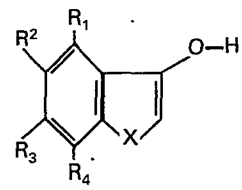

(II),

in which
$R_1$, $R_2$, $R_3$, $R_4$ and X have the above-given meaning, with amino acids and peptides of the general formula III

H–O–A–B (III),

in which
A and B have the above-given meaning,
or with reactive derivatives thereof.